# DEMANDE DE BREVET EUROPEEN

(11) **EP 0 877 075 A1**
(43) Date de publication de la demande: **11.11.1998**
(21) Numéro de dépôt: 98401045.4
(22) Date de dépôt: 29.04.1998
(51) Int. Cl.: C11D 1/66, C11D 3/22, A61L 2/00

(54) **Solution aqueuse d'entretien des lentilles de contact**

(30) Priorité: 05.05.1997 FR 9705489
(71) Demandeur: ESSILOR INTERNATIONAL (COMPAGNIE GENERALE D'OPTIQUE), F-94227 Charenton cédex (FR)
(72) Inventeur: Soyer, Patrice, 77390 Guignes (FR); Pochet, Arila, 77220 Gretz (FR)
(74) Mandataire: Casalonga, Axel

(57) **Abrégé**

La solution aqueuse d'entretien selon l'invention se caractérise par la présence d'une quantité effective de polyéthylène glycol 120 métflyl- glucosedioléate.

Application : aux solutions aqueuses d'entretien pour lentilles de contact rigides.

## Description

La présente invention concerne d'une manière générale une solution aqueuse d'entretien des lentilles de contact, en particulier des lentilles de contact rigides.

Les solutions aqueuses d'entretien des lentilles de contact ont généralement pour fonction de décontaminer et de nettoyer les lentilles.

Pour être efficaces, ces solutions doivent présenter une combinaison de plusieurs propriétés.

Tout d'abord, elles doivent assurer une décontamination effective des lentilles pour un large spectre de microorganismes et un nettoyage efficace des surfaces des lentilles, sans que leurs principes actifs soient absorbés par les lentilles.

D'autre part, pour simplifier l'entretien des lentilles, il est souhaitable que les lentilles puissent être réutilisées directement après le trempage dans la solution d'entretien, c'est-à-dire sans qu'il soit nécessaire d'effectuer un ou plusieurs rinçages à l'eau. Dans ce cas, il est particulièrement important, puisque la solution d'entretien doit entrer en contact avec l'oeil de l'utilisateur, que cette solution ne soit pas irritante, soit bien tolérée et rende confortable le port des lentilles pendant toute la durée d'utilisation continue, généralement une journée.

Un facteur important pour le confort d'utilisation des lentilles traitées est la viscosité de la solution d'entretien. Cette viscosité ne doit être ni trop faible, ni trop forte. Si la viscosité de la solution est trop faible, la solution ne mouillera pas convenablement les surfaces de la lentille ; celle-ci sera perçue par l'utilisateur comme un corps étranger dans l'oeil, et si cette viscosité est trop forte, la solution peut engendrer une vision trouble et conduire à une sensation de graisse sur la lentille, ce qui nuit au confort de l'utilisateur.

Un autre facteur important pour le confort d'utilisation de lentilles traitées avec une solution d'entretien est l'aptitude de la solution d'entretien à retenir sur les surfaces de la lentille traitée une quantité relativement importante d'eau, de telle sorte qu'il y ait une humidification pendant une durée prolongée des surfaces de la lentille et une rétention de la mouillabilité des surfaces de la lentille. Cette caractéristique, qui sera désignée ci-après comme "l'aptitude à la rétention de l'humidification" est un facteur important pour le confort de l'utilisateur. Cette aptitude à la rétention de l'humidification est un facteur particulièrement important dans le cas des lentilles de contact rigides qui généralement ont une faible aptitude à la rétention d'eau sur leurs surfaces.

Pour améliorer la mouillabilité des surfaces des lentilles de contact, en particulier des lentilles rigides, on a proposé d'incorporer dans les solutions d'entretien des monomères hydrophiles. Mais ces monomères hydrophiles ont en général l'inconvénient d'affecter les autres propriétés physiques des lentilles.

On a également proposé pour résoudre ce problème d'incorporer dans les solutions d'entretien des polymères mouillants, mais l'action de ces polymères est faible et disparaît rapidement.

Le document WO 95/00616 propose pour remédier aux inconvénients ci-dessus d'ajouter aux solutions d'entretien un poly(oxyde d'éthylène) soluble dans l'eau qui est un polymère étoile comportant un noyau hydrophobe ayant au moins trois atomes de carbone et au moins trois chaînes (bras) poly(oxyde d'éthylène) hydrophiles attachées au noyau. En outre, les polymères étoiles du document WO 95/00616 ne doivent pas comporter de bras hydrophobes attachés au noyau.

Dans une réalisation préférée du document WO 95/00616, un second composant polymère tensio-actif, un polymère cellulosique cationique, est ajouté à la solution d'entretien. Ce composant cationique se complexe avec le polymère étoile poly(oxyde d'éthylène) et le complexe formé est fortement adsorbé à la surface des lentilles.

Bien que les solutions d'entretien du document WO 95/00616 soient satisfaisantes, il serait souhaitable de disposer de solutions d'entretien assurant encore un meilleur confort à l'utilisateur des lentilles de contact.

La présente invention a donc pour objet de fournir une solution d'entretien pour lentilles de contact, en particulier pour lentilles de contact rigides, qui remédie aux inconvénients ci-dessus.

La présente invention a plus particulièrement pour objet une solution d'entretien telle que définie ci-dessus, qui confère aux lentilles traitées une aptitude améliorée à la rétention de l'humidification.

La présente invention a encore pour objet une solution d'entretien telle que définie ci-dessus qui soit d'utilisation simple, en particulier qui puisse permettre l'utilisation directe des lentilles traitées, c'est-à-dire sans rinçage, et assure à l'utilisateur un port confortable des lentilles traitées.

On atteint les buts énumérés ci-dessus, selon l'invention, en fournissant une solution aqueuse d'entretien pour lentilles de contact, en particulier des lentilles de contact rigides, caractérisée en ce qu'elle comprend une quantité effective de polyéthylèneglycol 120 méthylglucosedioléate de formule : 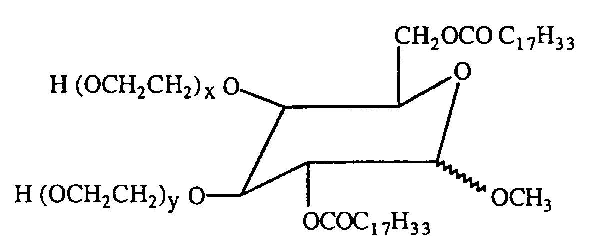 dans laquelle x + y = 120.

L'incorporation du polyéthylèneglycol 120 méthylglucosedioléate dans la solution d'entretien, non seulement accroît la mouillabilité des lentilles par la solution d'entretien, mais améliore notablement l'aptitude à la rétention de l'humidification des lentilles traitées. En outre, les solutions d'entretien selon l'invention assurent un confort particulièrement bon à l'utilisateur des lentilles traitées.

De préférence, la quantité de polyéthylèneglycol 120 méthylglucosedioléate présente dans la solution est comprise entre 0,05 et 5% en poids, mieux entre 0,1 et 2% en poids, par rapport au poids total de la solution aqueuse d'entretien.

Comme cela est bien connu, les compositions aqueuses d'entretien selon l'invention comprennent un agent de décontamination. Cet agent de décontamination, généralement un agent antimicrobien, est bien connu dans la technique et peut être constitué d'un seul composé ou d'un mélange de plusieurs composés. Les composés utiles comme agents de décontamination peuvent être tous composés connus utiles comme agents de décontamination dans les solutions aqueuses d'entretien des lentilles de contact.

Parmi ces composés, on peut citer la chlorhexidine (1,1'-hexaméthylène-bis[5-(p-chlorophényl)biguanide]) ou les sels hydrosolubles de celle-ci, tels que le gluconate de chlorhexidine; le polyhexaméthylène biguanide (un polymère d'hexaméthylène biguanide, également connu sous la dénomination polyaminopropyl biguanide) ou les sels hydrosolubles de celui-ci, tel que le chlorhydrate de polyhexaméthylène biguanide vendu sous la marque COSMOCIL CQ® (ICI Americas Inc.); les halogénures d'alkylammonium, en particulier les bromures d'alkyl triméthylammonium tels que le bromure de tétradécyl triméthylammonium, le bromure de dodécyl triméthylammonium, le bromure d'hexadécyl triméthylammonium (bromure de cétrimonium) parfois appelé "CETRIMIDE" ; les halogénures de benzalkonium tels que le chlorure de benzalkonium; les sels d'ammonium quaternaire polymères; et les mélanges de ces composés.

Les agents de décontamination recommandés selon l'invention sont le polyaminopropyl biguanide et les sels hydrosolubles de celui-ci, en particulier le produit commercialisé sous la marque COSMOCIL CQ®, les bromures d'alkylammonium, en particulier le bromure de cétrimonium et leurs mélanges.

Un agent de décontamination particulièrement préféré selon l'invention est un mélange de chlorhydrate de polyhexaméthylène biguanide (COSMOCIL CQ®) et de bromure de cétrimonium.

La quantité d'agent de décontamination dans la solution d'entretien selon l'invention est une quantité effective, c'est-à-dire une quantité suffisante pour assurer une décontamination des lentilles. En général, la quantité d'agent de décontamination présent dans la solution d'entretien selon l'invention est de 10⁻⁶ à 5% en poids, de préférence 0,01 à 0,05% en poids, par rapport au poids total de la solution d'entretien.

De préférence, les solutions d'entretien selon l'invention contiennent une quantité effective d'un ou plusieurs agents tensio-actifs non-ioniques. Parmi ces agents tensio-actifs non-ioniques, on peut citer les esters d'acides gras du polyéthylèneglycol, par exemple d'huile de noix de coco, les polysorbates, les polyoxyéthylène éthers et les polyoxypropylène éthers d'alcanes supérieurs (C₁₂-C₁₈). Des exemples des agents tensio-actifs non-ioniques ci-dessus comprennent le polysorbate 20 (commercialisé sous la marque TWEEN 20®), le polyoxyéthylène éther de lauryle (BRIJ® 35), le stéarate de polyoxyéthylène (40) (MYRJ® 52), le stéarate de polyoxyéthylène (25) propylèneglycol (ATLAS® G 2612).

Une classe particulièrement recommandée d'agents tensio-actifs non-ioniques comprend les poly(oxypropylène)-poly(oxyéthylène) adducts de l'éthylènediamine et les polymères séquencés poly(oxyéthylène)-poly(oxypropylène).

Les agents tensio-actifs non-ioniques préférés sont les polymères séquencés poly(oxyéthylène)-poly(oxypropylène).

Les solutions d'entretien selon l'invention contiennent en général de 0,01 à 15% en poids d'agent tensio-actif non-ionique par rapport au poids total de la composition, de préférence de 0,05 à 1% en poids.

De préférence encore, l'agent tensio-actif non-ionique polymère séquencé poly(oxyéthylène)-poly(oxypropylène) recommandé est présent dans la solution d'entretien en une quantité telle que le rapport pondéral entre cet agent tensio-actif non-ionique recommandé et le polyéthylèneglycol 120 méthylglucose dioléate soit d'environ 1:2.

Bien que cela ne soit pas préféré, la solution d'entretien peut également comprendre des agents tensio-actifs cationiques et/ou amphotères classiquement utilisés dans les compositions d'entretien de lentilles de contact dans les proportions usuelles.

Les solutions d'entretien selon l'invention comprennent de préférence en outre un ou plusieurs agents classiques de réglage de la viscosité. Ces agents de réglage de la viscosité sont bien connus et comprennent les polymères de cellulose hydrosolubles tels l'hydroxyéthyl ou l'hydroxypropylcellulose, la carboxyméthylcellulose, la polyvinylpyrrolidone et les poly(acide acrylique). Les agents de réglage de la viscosité préférés sont les polymères de cellulose et en particulier l'hydroxyéthylcellulose.

L'agent de réglage de la viscosité est utilisé dans les quantités usuelles, généralement de 0,01 à 4,0% en poids, ou moins, par rapport au poids total de la solution.

Outre le polyéthylèneglycol 120 méthylglucose dioléate, les solutions d'entretien selon l'invention contiennent généralement d'autres agents mouillants classiquement utilisés dans les solutions d'entretien des lentilles de contact, tels que les poly(oxyéthylène) glycol. Ces agents mouillants sont généralement présents à raison de 0,5 à 5%, de préférence 1 à 2% en poids par rapport au poids total de la solution.

De préférence encore, l'agent mouillant de type poly(oxyéthylène) glycol est présent dans la solution d'entretien en une quantité telle que le rapport pondéral entre cet agent mouillant recommandé et le polyéthylèneglycol 120 méthylglucose dioléate soit d'environ 1:1.

En général, les solutions d'entretien selon l'invention comprennent un ou plusieurs agents séquestrants (ou chélatant), en particulier des ions calcium et magnésium, en quantité pouvant atteindre jusqu'à environ 2% en poids par rapport au poids total de la solution. Parmi les agents séquestrants utilisables dans les solutions de l'invention, on peut citer l'acide éthylène diamine tétraacétique (EDTA) et ses sels, en particulier son sel de sodium, les complexes de polyphosphate tels que l'hexamétaphosphate de sodium, le pyrophosphate de sodium et le tripolyphosphate de sodium, l'acide gluconique, l'acide citrique, l'acide tartrique et leurs sels, en particulier leurs sels de sodium. L'agent séquestrant préféré est le sel de sodium de l'EDTA.

Les solutions d'entretien de l'invention contiennent encore généralement un agent tampon. Parmi les tampons utilisables dans la solution d'entretien selon l'invention, on peut citer les phosphates tels que Na₂H PO₄, NaH₂PO₄ et K H₂PO₄, les tampons borates tels que l'acide borique, le borate de sodium, le tétraborate de potassium, le métaborate de potassium ou des mélanges de ces tampons. Un tampon recommandé est un mélange de phosphate monosodique et de phosphate disodique. La quantité d'agent tampon utilisé est comprise en général entre 0,05 et 2,5% en poids, de préférence entre 1 et 2% en poids par rapport au poids total de la solution.

Généralement, la tonicité des solutions aqueuses d'entretien selon l'invention est ajustée par addition d'un agent de tonicité classique, tel que du chlorure de sodium ou une solution de glycérol. L'agent de tonicité préféré est le chlorure de sodium.

Les solutions d'entretien selon l'invention peuvent encore comporter dans les quantités usuelles, tous autres ingrédients classiquement utilisés dans les solutions d'entretien de lentilles de contact.

A titre d'exemple d'un autre ingrédient qui peut être incorporé aux solutions d'entretien selon l'invention, on peut citer des agents germicides tels que le thimerosal, l'acide sorbique, le 1,5-pentanediol, les sels phényl mercuriques d'alkyl thioéthanolamines. De tels agents germicides supplémentaires sont décrits dans le document EP-0 180 309.

Bien que dans la description qui précède, on se réfère principalement à des solutions de décontamination et de nettoyage, le composé polyéthylèneglycol 120 méthylglucose dioléate peut être avantageusement utilisé dans d'autres solutions d'entretien de lentilles de contact, telles que des solutions de conservation et de trempage.

### EXEMPLES

On a préparé une solution aqueuse d'entretien selon l'invention, ayant la composition suivante :
- Polyaminopropyl biguanide (COSMOCIL CQ® solution à 20%) (soit 0,0002 g de principe actif) 0,001 g
- Bromure de cétrimonium 0,015 g
- Hydroxyéthyl cellulose 0,500 g
- Polyéthylèneglycol 120 méthylglucose dioléate 1,200 g
- Polymère séquencé poly(oxyéthylène)-poly(oxypropylène) (Poloxamer) 0,500 g
- EDTA disodique 0,065 g
- Polyoxyéthylèneglycol (Macrogol) 1,200 g
- Tampon de phosphate monosodique, 2 H₂O et Phosphate disodique, 12 H₂O 2,050 g
- Chlorure de sodium 0,160 g
- Eau déminéralisée qsp 100 ml

On a comparé la solution d'entretien ci-dessus avec des solutions d'entretien du commerce. En particulier, on a déterminé l'aptitude à la rétention de l'humidification des solutions en procédant de la manière suivante.

On pèse des lentilles de contact rigides à sec puis on les trempe dans chacune des solutions testées, on les ressort et on les égoutte. On repèse chacune des lentilles et on en déduit la quantité de chaque solution déposée sur chaque lentille. On laisse les solutions déposées s'évaporer pendant deux heures, on repèse les lentilles et on détermine la quantité de composants restants sur les lentilles. La différence entre le poids de solution initialement déposée sur les lentilles et le poids des composants restants sur les lentilles après 2 heures d'évaporation, est une indication de l'aptitude de la solution à la rétention de l'humidification.

**TABLEAU I**

| | | Solutions d'entretien du commerce | | |
|---|---|---|---|---|
| | Exemple 1 | BOSTON® SIMPLICITY | SOLOCARE® HARD | TOTALCARE® |
| pH | 7,20 | 7,26 | 7,51 | 7,10 |
| | | | | |
| Viscosité-Pa.s x 10⁻³ (LVI 30 t/min à 20°C) | 10 | 31 | 15 | 58 |
| | | | | |
| Densité | 1,016 | 1,010 | 1,007 | 1,010 |
| | | | | |
| Poids de solution restant sur la lentille après trempage et égouttage (g) | 0,0067 | 0,0048 | 0,0042 | 0,0048 |
| | | | | |
| Poids des composants restant sur la lentille après évoporation de la solution (g) | 0,0006 | 0,0002 | 0,0008 | 0,0005 |
| | | | | |
| ΔP | 0,0061 | 0,0046 | 0,0034 | 0,0043 |

Les résultats montrent que la solution d'entretien de l'exemple 1 présente une amélioration de l'aptitude à la rétention de l'humidification de 32,6%, 79,4% et 41,8% par rapport aux solutions du commerce.

On a déterminé la tolérance oculaire conférée par différentes solutions selon l'invention ainsi que des solutions "à blanc", et des solutions selon les exemples 6C et 14A du document WO 95/00616.

Outre la solution de l'exemple 1, les solutions testées étaient les suivantes :
- Exemple comparatif A : solution de l'exemple 1 sans Glucamate DOE 120
- Exemple comparatif B : solution de l'exemple 1 avec 0,1% en poids de Glucam® E20 à la place du Glucamate DOE 120
- Exemple comparatif C : solution de l'exemple 1 avec 1,2% en poids de Glucam® E 20 à la place du Glucamate DOE 120
- Exemple comparatif D : solution de l'exemple 1 avec 3% en poids de Glucam® E 20 à la place du Glucamate DOE 120
- Exemple comparatif E : solution de l'exemple 6C du document WO 95/00616 (0,3% Glucam® E 20)
- Exemple 2 : solution de l'exemple 6C du document WO 95/00616 dans laquelle on a remplacé le Glucam® E 20 par 0,3% en poids de Glucamate DOE 120
- Exemple 3 : solution de l'exemple 6C du document WO 95/00616 dans laquelle on a remplacé le Glucam® E 20 par 1,2% en poids de Glucamate DOE 120
- Exemple comparatif F : solution de l'exemple 14A du document WO 95/00616 sans Glucam® E 20 (contient un tensio-actif cationique - Polymère JR-30M)
- Exemple comparatif G : solution de l'exemple 14A du document WO 95/00616 avec 0,02% en poids de Glucam® E 20
- Exemple 4 : solution de l'exemple 14A du document WO 95/00616 dans lequel on a remplacé le Glucam® E 20 par 0,02% en poids de Glucamate DOE 120.

La tolérance oculaire a été déterminée par instillation directe des solutions dans les yeux d'un testeur auquel on a demandé s'il ressentait une sensation de gêne oculaire.

Les résultats sont indiqués dans le tableau II ci-dessous.

**TABLEAU II**

| Exemple n° | Tolérance oculaire |
|---|---|
| A | X |
| B | X |
| C | X |
| D | X |
| E | X |
| F | X |
| G | X |
| 1 | O |
| 2 | O |
| 3 | O |
| 4 | O |
| O pas de sensation de gêne oculaire. | |
| X sensation de gêne oculaire. | |

La solution de l'exemple 1 a été utilisée par 80 porteurs de lentilles de contact pendant une période de 180 jours, avec un suivi médical.

Les observations biomicroscopiques répétées des yeux des porteurs n'ont révélé aucune manifestation d'intolérance à la solution.

De plus, les résultats quant à la mouillabilité, aux rayures et aux dépôts protéiniques observés par les investigateurs à la surface des lentilles montrent que celles-ci sont bien préservées par la solution d'entretien.

Enfin, aux questions relatives à l'efficacité d'entretien, au confort à l'insertion des lentilles, au confort de fin de journée et à la simplicité d'utilisation de la solution d'entretien de l'exemple 1, 96% des sujets testés se déclarent satisfaits.

L'addition de polyéthylèneglycol 120 méthylglucose dioléate aux solutions d'entretien de lentilles de contact, en particulier des lentilles de contact rigides, conduit à des solutions conférant un meilleur confort d'utilisation des lentilles grâce notamment à l'amélioration de l'aptitude à la rétention de l'humidification sur les lentilles qu'apportent ces solutions.

La combinaison de polyéthylèneglycol 120 méthylglucose dioléate et d'un agent tensio-actif non-ionique s'avère particulièrement avantageuse pour l'obtention d'une solution d'entretien mieux tolérée par l'utilisateur.

## Revendications

1. Solution aqueuse d'entretien de lentilles de contact, caractérisée en ce qu'elle comprend une quantité effective de polyéthylèneglycol 120 méthylglucosedioléate de formule : 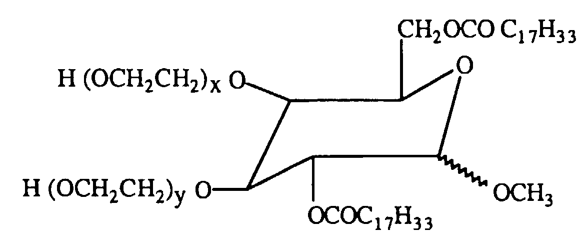 dans laquelle x + y = 120.

2. Solution aqueuse d'entretien selon la revendication 1, caractérisée en ce que la quantité de polyéthylèneglycol 120 méthylglucosedioléate est comprise entre 0,05 et 5% en poids par rapport au poids total de la solution.

3. Solution aqueuse d'entretien selon la revendication 1 ou 2, caractérisée en ce qu'elle comprend un agent de décontamination choisi parmi : la chlorhexidine et les sels hydrosolubles de celle-ci, le polyhexaméthylène biguanide et les sels hydrosolubles de celui-ci, les halogénures d'alkylammonium, les halogénures de benzalkonium, les sels d'ammonium quaternaire et leurs mélanges.

4. Solution aqueuse d'entretien selon la revendication 3, caractérisée en ce que l'agent de décontamination est un mélange de polyhexaméthylène biguanide ou d'un sel hydrosoluble de celui-ci et d'un ou plusieurs halogénures d'alkylammonium.

5. Solution aqueuse d'entretien selon la revendication 4, caractérisée en ce que l'halogénure d'alkylammonium est le bromure de cétrimonium.

6. Solution aqueuse d'entretien selon l'une quelconque des revendications 3 à 5, caractérisée en ce que l'agent de décontamination représente de 10⁻⁶ à 5%, de préférence 0,01 à 0,05% en poids, par rapport au poids total de la solution.

7. Solution aqueuse d'entretien selon l'une quelconque des revendications précédentes, caractérisée en ce qu'elle comprend un ou plusieurs agents tensio-actifs choisis parmi les agents tensio-actifs non-ioniques, cationiques et amphotères.

8. Solution aqueuse d'entretien selon la revendication 7, caractérisée en ce que l'agent tensio-actif présent est uniquement non-ionique.

9. Solution aqueuse d'entretien selon la revendication 7 ou 8, caractérisée en ce que l'agent tensio-actif non-ionique est un polymère séquencé poly(oxyéthylène)-poly(oxypropylène).

10. Solution aqueuse d'entretien selon l'une quelconque des revendications 7 à 9, caractérisée en ce que l'agent tensio-actif représente 0,01 à 15%, de préférence 0,05 à 1%, en poids par rapport au poids total de la composition.

11. Solution aqueuse d'entretien selon l'une quelconque des revendications précédentes, caractérisée en ce qu'elle comprend un agent de réglage de la viscosité choisi parmi les polymères de cellulose hydrosolubles.

12. Solution aqueuse d'entretien selon l'une quelconque des revendications précédentes, caractérisée en ce qu'elle comprend un agent séquestrant choisi parmi l'acide éthylène diamine tétraacétique, l'acide gluconique, l'acide citrique, l'acide tartrique, les sels de ces acides et les complexes de polyphosphate.

13. Solution aqueuse d'entretien selon l'une quelconque des revendications précédentes, caractérisée en ce qu'elle comprend un tampon choisi parmi les tampons phosphate, les tampons borate et leurs mélanges.

14. Solution aqueuse d'entretien selon l'une quelconque des revendications précédentes, caractérisée en ce qu'elle comprend un agent de tonicité.
